# EUROPÄISCHE PATENTSCHRIFT

(11) **EP 0 861 822 B1**
(45) Veröffentlichungstag und Bekanntmachung des Hinweises auf die Patenterteilung: **04.07.2001**
(21) Anmeldenummer: 98100937.6
(22) Anmeldetag: 21.01.1998
(51) Int. Cl.: C07C 67/08, C07C 67/293, C07C 69/007, C07C 69/78, C07C 69/145

(54) **Verfahren zur Herstellung von 1-funktionellen Allyalkohol-Carbonsäureestern**
Process for the preparation of carboxylic acid esters of 1-functional allyl alcohols
Procédé de préparation d'esters d'acides carboxyliques d'alcools allyliques 1-fonctionnels

(30) Priorität: 27.02.1997 DE 19707959
(43) Veröffentlichungstag der Anmeldung: 02.09.1998
(73) Patentinhaber: Dragoco Gerberding & Co Aktiengesellschaft, 37603 Holzminden (DE)
(72) Erfinder: Vollhardt, Jürgen, Dr., 37639 Bevern (DE)
(74) Vertreter: Eisenführ, Speiser & Partner

(56) Entgegenhaltungen:
- DE-A- 2 513 198
- DATABASE WPI Week 7552 Derwent Publications Ltd., London, GB; AN 75-85552w XP002066191 & JP 50 116 411 A (TORAY IND INC) , 11.September 1975

## Beschreibung

Die Gruppe der 1-funktionellen Allylalkohol-Carbonsäureester umfaßt wirtschaftlich bedeutende Verbindungen, die entweder als solche nutzbar sind oder zu weiteren wirtschaftlich bedeutenden Verbindungen umgesetzt, beispielsweise zu den entsprechenden 1-funktionellen Allylalkoholen verseift werden können.

Es ist bekannt, daß 1-funktionelle Allylalkohol-Carbonsäureester der allgemeinen Formel 2 gemäß dem nachfolgenden Schema mit der sogenannten 3,1-Allylumlagerung 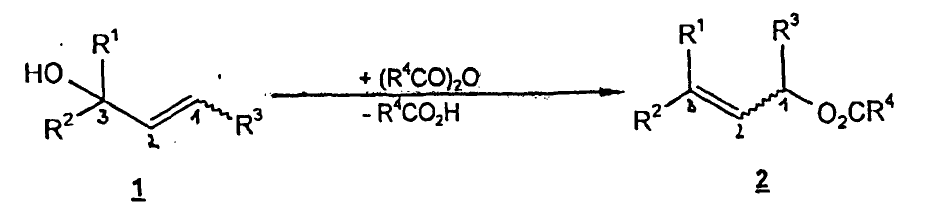
- R¹ und R³ =: H, Alkyl oder Aryl
- R² =: beliebiger Rest, aber kein H
- R⁴ =: Alkyl oder Aryl
durch Umsetzung von 3-funktionellen sekundären oder tertiären Allylalkoholen der allgemeinen Formel 1 mit Carbonsäureanhydriden erhalten werden können. Die 3-funktionellen Allylalkohole der allgemeinen Formel 1 sind ihrerseits duch Addition von Vinyl- oder Acetylenmetallverbindungen an Carbonylverbindungen zugänglich.

Diese Umlagerungsreaktion, bei der gleichzeitig eine Veresterung der umgelagerten Alkoholfunktion stattfindet, verläuft jedoch bislang insbesondere hinsichtlich der erzielten Ausbeuten recht unbefriedigend. In einem typischen Beispiel wird sie unter Anwendung stark saurer Bedingungen in einem Lösungsmittelgemisch aus Essigsäure, Acetanhydrid und p-Toluolsulfonsäure durchgeführt, wobei die Acetate der umgelagerten Allylalkohole erhalten werden. Dabei kommt es jedoch zu unerwünschten Nebenreaktionen, wie z.B. Eliminierungen oder Cyclisierungen, die die Ausbeute an gewünschtem umgelagerten Produkt stark schmälern. So werden beim Einsatz von z.B. Linalool hauptsächlich Cyclisierungen beobachtet, während die gewünschten Produkte Geraniolacetat und Nerolacetat nur als Nebenkomponenten entstehen.

Mit der Erfindung soll das Verfahren dahingehend verbessert werden, daß sich mit wenig Aufwand erheblich verbesserte Ausbeuten einstellen. Dieses Ziel wird dadurch erreicht, daß der 3-funktionelle Allylalkohol in Gegenwart einer Verbindung oder mehrerer Verbindungen eines Metalls oder mehrerer Metalle der V. bis VII. Nebengruppe bei einer Temperatur oberhalb von 100 °C mit dem Anhydrid der Carbonsäure umgesetzt wird. überraschend wurde festgestellt, daß auf diese Weise in ca. 1 - 5 h eine Ausbeute von mehr als 70 %, meistens bis zu ca. 80 % an dem Allylalkohol-Carbonsäureester der allgemeinen Formel 2 erzielen läßt.

Die Verwendung von Übergangsmetall-Katalysatoren bei der Allylumlagerung ist an sich bekannt. So werden gemäß der US-Patentschrift 3,925,485 (*Chabardes et al*) 3-funktionelle Allylalkohole bei höheren Temperaturen in Gegenwart von Vanadium (V)-Verbindungen zu den isomeren 1-funktionellen Allylalkoholen umgesetzt, und gemäß der deutschen Offenlegungsschrift DE 25 16 698 A1 (*Hosogai et al*) erfolgt eine ähnliche Umsetzung in Gegenwart von Wolframsäureestern. In beiden Fällen wird jedoch kein 1-funktioneller Allylalkohol-Carbonsäureester gebildet, sondern der 1-funktionelle Alkohol, der mit dem 3-funktionellen Alkohol im Gleichgewicht steht. Die Gleichgewichtslage liegt dabei im allgemeinen auf der Eduktseite, d. h. auf der Seite des höhersubstituierten Alkohols. Bei der Umlagerung von Linalool werden z. B. nur ca. 30 - 40 % Geraniol und Nerol als Gemisch gebildet. Das Reaktionsprodukt kann darüberhinaus nicht aus dem Reaktionsgemisch durch Abdestillieren entfernt werden, um so einen vollständigen Umsatz zu erzielen, wenn es wie z.B. Geraniol/Nerol einen höheren Siedepunkt als das Edukt aufweist. Die Reaktion erreicht deshalb nur eine Endausbeute von ca. 30 - 40 % und muß dann abgebrochen werden. Demgegenüber war nicht vorhersehbar, daß sich bei erfindungsgemäßer Reaktionsführung gut doppelt so hohe Ausbeuten ergeben.

Als Metallverbindungen werden vorzugsweise die Oxide und Hydroxide (Metallsäuren) der übergangsmetalle in der höchsten oder zweithöchsten Wertigkeitsstufe eingesetzt. Geringere Wertigkeitsstufen sind nicht grundsätzlich ausgeschlossen, führen im allgemeinen aber zu minder guten Ausbeuten. Gute Ergebnisse werden mit Wolframsäure bzw. deren Salzen und/oder Wolfram(VI)oxid und/oder Molybdän(V)oxid und/oder Molybdän(VI)oxid und/oder Rhenium(VII)oxid erreicht. Aus der Gruppe der genannten Metallverbindungen ragen die Verbindungen des Wolframs noch heraus, denn bei ihrem Einsatz verläuft die Reaktion entweder schneller oder bezüglich unerwünschter Oxidations-Nebenreaktionen selektiver als bei (alleinigem) Einsatz der anderen genannten Metallverbindungen.

Zweckmäßig ist es, dem Reaktionsgemisch noch eine ausreichende Menge eines Alkalisalzes einer schwachen Säure, z.B. Natriumcarbonat oder Natriumacetat zuzusetzen, um sicherzustellen, daß im Reaktionsgemisch möglichst keine freien Protonen enthalten sind. Bei Einsatz einer freien Metallsäure ist der Zusatz eines solchen Salzes sehr geboten, aber auch bei Einsatz anderer Metallverbindungen wie Oxiden oder Salzen der Metallsäuren empfehlenswert.

Der 3-funktionelle Allylalkohol und das Carbonsäureanhydrid sollten mindestens in äquimolaren Mengen eingesetzt werden, wobei ein geringer Überschuß an Anhydrid aus reaktionskinetischen Gründen bevorzugt ist. Es kann auch mit Unterschuß an Anhydrid gearbeitet werden, aber dann stellt sich keine vollständige Umsetzung ein, und dementsprechend vermindern sich die Ausbeuten.

Die nachfolgenden Beispiele erläutern die Erfindung:

### Beispiel 1: Umsetzung von Linalool mit Acetanhydrid und Wolframsäure/Natriumacetat

Zu einer Mischung aus 561 g Acetanhydrid (5,5 mol), 7,5 g Natriumacetat (91,5 mmol) und 2,0 g Wolframsäure (8,0 mmol) werden bei 130 - 140 °C 770 g (5,0 mol) Linalool zugetropft. Nach insgesamt 2 h wird bei 80° C Wasser zugegeben. Es entstehen zwei Phasen, die voneinander getrennt werden. Die organische Phase wird zweimal mit Wasser ausgewaschen. Es entstehen 870 g eines gelblichen Öles mit einem Gehalt an Geranylacetat von ca. 50 % und Nerylacetat von ca. 25 % sowie ca. 1,5 % Terpineolacetat (Analyse durch Gas-Chromatographie).

### Beispiel 2: Umsetzung von Linalool mit Benzoesäureanhydrid und Wolframsäure/Natriumcarbonat

Zu einer Mischung aus 113 g Benzoesäureanhydrid (0,5 mol), 1,0 g Natriumcarbonat (9,4 mmol) und 1,1 Wolframsäure (4,4 mmol) werden bei 130 - 140 °C 70 g (0,45 mol) Linalool zugetropft. Nach insgesamt 4,5 h wird bei 80 °C Wasser und Toluol zugegeben. Es entstehen zwei Phasen die voneinander getrennt werden. Die organische Phase wird mit Wasser und Sodalösung ausgewaschen. Das Lösungsmittel wird abdestilliert. Es entstehen 110 g eines gelblichen Feststoffs mit einem Gehalt an Geranylbenzoat von ca. 53 % und Nerylbenzoat von ca. 30 % (Analyse durch Gas-Chromatographie).

### Beispiel 3: Umsetzung von 6,7-Dihydrolinalool mit Acetanhydrid und Wolframsäure/Natriumacetat

Zu einer Mischung aus 29 g Acetanhydrid (0,28 mol), 0,3 g Natriumacetat (3,6 mmol) und 0,1 g Wolframsäure (0,4 mmol) werden bei 130 - 140 °C 39 g (0,25 mol) 6,7-Dihydrolinalool zugetropft. Nach insgesamt 1 wird bei 80 °C Wasser und Toluol zugegeben. Es entstehen zwei Phasen, die voneinander getrennt werden. Die organische Phase wird mit Wasser ausgewaschen. Das Lösungsmittel wird abdestilliert. Es entstehen 48 g eines gelblichen Öles mit einem Gehalt an Dihydrogeranylacetat von ca. 54 % und Dihydronerylacetat von ca. 28 % (Analyse durch Gas-Chromatographie).

### Beispiel 4: Umsetzung von Linalool mit Acetanhydrid und Holybdän(VI)-oxid/Natriumacetat

Zu einer Mischung aus 12 g Acetanhydrid (118 mmol), 0,5 g Natriumacetat (6,1 mmol) und 0,1 g Molybdän(VI)-oxid (0,69 mmol) werden bei 130 - 140 °C 13,8 g (90 mmol) Linalool zugetropft. Nach insgesamt 2,5 h wird bei 80 °C Wasser zugegeben. Es entstehen zwei Phasen, die voneinander getrennt werden. Die organische Phase wird zweimal mit Wasser ausgewaschen. Es können ca. 20 g eines gelblichen öles isoliert werden. Nach Analyse durch Gas-Chromatographie hat sich Linalool zu 72 % in ein Gemisch aus Geranyl- und Nerylacetat umgewandelt.

### Beispiel 5: Umsetzung von Linalool mit Acetanhydrid und Rhenium(VII)-oxid/Natriumacetat

Zu einer Mischung aus 12 g Acetanhydrid (118 mmol), 0,5 g Natriumacetat (6,1 mmol) und 0,1 g Rhenium(VII)-oxid (0,2 mmol) werden bei 130 - 140 °C 13,8 g (90 mmol) Linalool zugetropft. Nach insgesamt einer Stunde wird bei 80 °C Wasser zugegeben. Es entstehen zwei Phasen, die voneinander getrennt werden. Die organische Phase wird zweimal mit Wasser ausgewaschen. Es können ca. 20 g eines gelblichen öles isoliert werden. Nach Analyse durch Gas-Chromatographie hat sich Linalool zu 82 % in ein Gemisch aus Geranyl- und Nerylacetat umgewandelt.

### Beispiel 6: Umsetzung von 1-Octen-3-ol mit Acetanhydrid und Wolframsäure

Zu einer Mischung aus 112,2 g Acetanhydrid (1,1 mmol), 6,6 g Natriumacetat (80 mmol) und 2,5 g Wolframsäure (10 mmol) werden bei 130 - 140 °C 128 g (1 mol) 1-Octen-3-ol zugetropft. Nach insgesamt einer Stunde wird bei 80 °C Wasser zugegeben. Es entstehen zwei Phasen, die voneinander getrennt werden. Die organische Phase wird zweimal mit Wasser ausgewaschen. Es können ca. 140 g eines gelblichen öles isoliert werden. Nach Analyse durch Gas-Chromatographie hat sich 1-Octen-3-ol zu 75 % in 2-Octen-1-ylacetat umgewandelt.

## Patentansprüche

1. Verfahren zur Herstellung von 1-funktionellen Allylalkohol-Carbonsäureestern der allgemeinen Formel 2, 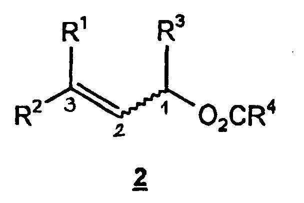 wobei ein 3-funktioneller Allylalkohol der allgemeinen Formel 1 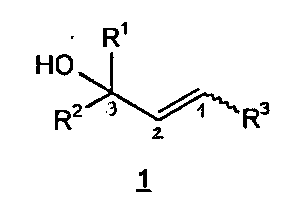 in Gegenwart einer Verbindung oder mehrerer Verbindungen eines Metalls oder mehrerer Metalle der V. bis VII. Nebengruppe bei einer Temperatur oberhalb von 100° C mit dem Anhydrid (R⁴CO)₂O einer Carbonsäure umgesetzt wird, wobei
R¹ und R³ = H, Alkyl oder Aryl
R² = beliebiger Rest, aber kein H
R⁴ = Alkyl oder Aryl.

2. Verfahren nach Anspruch 1, wobei die Umsetzung bei einer Temperatur von über 100 bis zu 200° C, vorzugsweise 130 - 150° C, durchgeführt wird.

3. Verfahren nach Anspruch 1 oder 2, wobei eine oder mehrere oxidische Metallverbindungen eingesetzt werden.

4. Verfahren nach einem der Ansprüche 1 - 3, wobei ein oder mehrere Metalle der V. bis VII. Nebengruppe in der höchsten oder zweithöchsten Wertigkeitsstufe vorliegen.

5. Verfahren nach einem der Ansprüche 1 - 4, wobei Wolframsäure bzw. deren Salze und/oder Wolfram(VI)oxid und/oder Molybdän(V)oxid und/oder Molybdän(VI)oxid und/oder Rhenium(VII)oxid eingesetzt werden.

6. Verfahren nach einem der vorhergehenden Ansprüche, wobei das Carbonsäureanhydrid und der 3-funktionelle Allylalkohol in äquimolaren Mengen eingesetzt werden oder ein geringer Überschuß an Carbonsäureanhydrid eingesetzt wird.

7. Verfahren nach einem der vorhergehenden Ansprüche, wobei dem Reaktionsgemisch eine zum Abfangen freier Protonen ausreichende Menge eines Alkalisalzes einer schwachen Säure, wie Natriumcarbonat oder Natriumacetat, zugesetzt wird.

## Claims

1. Process for preparing 1-functional allyl alcohol carboxylic esters of the general formula 2, 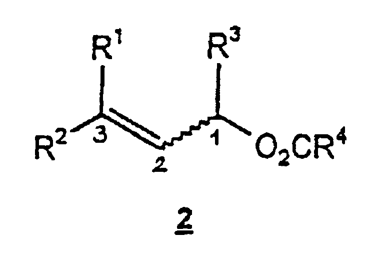 in which a 3-functional allyl alcohol of the general formula 1 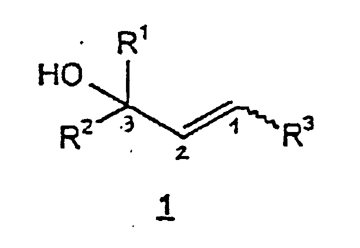 is reacted with the anhydride (R⁴CO)₂O of a carboxylic acid in the presence of one or more compounds of one or more metals of transition groups V to VII at a temperature above 100°C, where
R¹ and R³ = H, alkyl or aryl
R² = any radical, but not H
R⁴ = alkyl or aryl.

2. Process according to Claim 1, in which the reaction is carried out at a temperature of > 100 to 200°C, preferably 130-150°C.

3. Process according to Claim 1 or 2, in which one or more oxidizing metal compounds are used.

4. Process according to any of Claims 1 to 3, in which one or more of the metals of transition groups V to VII lie in the highest or second highest oxidation state.

5. Process according to any of Claims 1 to 4, in which tungstic acid or its salts and/or tungsten(VI) oxide and/or molybdenum(V) oxide and/or molybdenum(VI) oxide and/or rhenium(VII) oxide is/are used.

6. Process according to any of the preceding claims, in which the carboxylic anhydride and the 3-functional allyl alcohol are used in equimolar amounts or in a small excess of carboxylic anhydride.

7. Process according to any one of the preceding claims, in which an alkali salt of a weak acid, such as sodium carbonate or sodium acetate, is added to the reaction mixture in an amount sufficient to neutralize free protons.

## Revendications

1. Procédé de préparation d'esters d'acides carboxyliques d'alcools allyliques 1-fontionnels de formule générale 2, 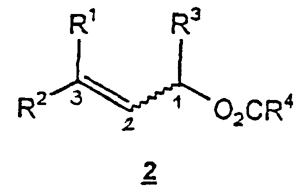 dans lequel on fait réagir un alcool allylique 3-fonctionnel de formule générale 1 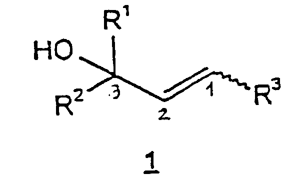 dans lesquelles :
R¹ et R³ = H, un alkyle ou un aryle,
R² = un reste quelconque, mais pas H,
R⁴ = un alkyle ou un aryle,
avec de l'anhydride (R⁴CO)₂O d'un acide carboxylique en présence d'un composé ou de plusieurs composés d'un métal ou de plusieurs métaux du groupe V à VII, à une température supérieure à 100 °C.

2. Procédé suivant la revendication 1, dans lequel on réalise la réaction à une température supérieure à 100 °C jusqu'à 200 °C, de préférence comprise entre 130 °C et 150 °C.

3. Procédé suivant la revendication 1 ou 2, dans lequel on introduit un ou plusieurs combinés métalliques oxydés.

4. Procédé suivant une des revendications 1 à 3, dans lequel un ou plusieurs métaux du groupe V à VII se présentent avec un état de valence le plus élevé ou immédiatement inférieur.

5. Procédé suivant une des revendications 1 à 4, dans lequel on introduit des acides de tungstène ou bien leurs sels et/ou de l'oxyde de tungstène (VI) et/ou de l'oxyde de molybdène (V) et/ou de l'oxyde de molybdène (VI) et/ou de l'oxyde de rhénium (VII).

6. Procédé suivant une des revendications précédentes, dans lequel on introduit l'anhydride d'acide carboxylique et l'alcool allylique 3-fonctionnel en quantités équimolaires ou avec un peu d'excès d'anhydride d'acide carboxylique.

7. Procédé suivant une des revendications précédentes, dans lequel, au mélange réactionnel, on ajoute une quantité, suffisante pour la fixation de protons libres, d'un sel alcalin d'un acide faible, tel que du carbonate de sodium ou de l'acétate de sodium.
